(19) **Europäisches Patentamt / European Patent Office / Office européen des brevets**

(11) **EP 4 138 695 B1**

(12) **EUROPEAN PATENT SPECIFICATION**

(45) Date of publication and mention of the grant of the patent:
**05.11.2025 Bulletin 2025/45**

(51) International Patent Classification (IPC):
**A61B 18/14** (2006.01)    **A61B 18/00** (2006.01)
**A61B 5/00** (2006.01)    **A61B 18/12** (2006.01)

(21) Application number: **21724130.6**

(52) Cooperative Patent Classification (CPC):
**A61B 18/148; A61B 18/1482; A61B 18/1485;**
**A61B 18/1492;** A61B 5/7221; A61B 18/1233;
A61B 2018/00577; A61B 2018/00613;
A61B 2018/00642; A61B 2018/00702;
A61B 2018/00708; A61B 2018/00839

(22) Date of filing: **20.04.2021**

(86) International application number:
**PCT/US2021/028108**

(87) International publication number:
**WO 2021/216515 (28.10.2021 Gazette 2021/43)**

(54) **ADAPTIVE ECG TRIGGERING IN AN ABLATION SYSTEM**

ADAPTIVE EKG-AUSLÖSUNG IN EINEM ABLATIONSSYSTEM

DÉCLENCHEMENT D'ECG ADAPTATIF DANS UN SYSTÈME D'ABLATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.04.2020 US 202063013161 P**

(43) Date of publication of application:
**01.03.2023 Bulletin 2023/09**

(73) Proprietor: **Boston Scientific Scimed, Inc.**
**Maple Grove, MN 55311 (US)**

(72) Inventors:
• **GORZYCKI, Jonathan Tyler**
  **Blaine, Minnesota 55449 (US)**
• **CAO, Hong**
  **Maple Grove, Minnesota 55311 (US)**
• **FORSYTH, Bruce R.**
  **Hanover, Minnesota 55341 (US)**
• **CANADY, Larry D., Jr.**
  **Ham Lake, Minnesota 55304 (US)**
• **OSTROOT, Timothy A.**
  **Cokato, Minnesota 55321 (US)**

(74) Representative: **Vossius & Partner**
**Patentanwälte Rechtsanwälte mbB**
**Siebertstraße 3**
**81675 München (DE)**

(56) References cited:
**WO-A1-2018/005511**    **WO-A2-2015/171921**
**US-A1- 2016 166 310**

**Description**

BACKGROUND

Removal or destruction of diseased tissue is a goal of many cancer treatment methods. Tumors may be surgically removed, however, less invasive approaches gamer much attention. Tissue ablation is a minimally invasive method of destroying undesirable tissue in the body. A variety of ablation techniques have been developed, many using the application of electricity or other energy via a probe placed on or inserted into or adjacent target tissue. For example, heat-based thermal ablation adds heat to destroy tissue, while cold ablation does the opposite, resulting in cell death by the application of cold temperatures. Radio-frequency (RF) thermal, microwave and high intensity focused ultrasound ablation can each be used to raise localized tissue temperatures well above the body's normal 37 degrees C. Irreversible electroporation (IRE) uses electric fields to expand pores in the cell membrane beyond the point of recovery, causing cell death for want of a patent cell membrane. IRE typically uses a narrower pulse width than RF ablation to reduce thermal effects.

With the application of an electrical signal for ablation purposes, there is a small risk of interfering with the cardiac cycle. More particularly, a voltage applied to a patient during a vulnerable period of the cardiac cycle can trigger an arrhythmia. For example, cardiac stimulus applied during the T-wave poses a risk of inducing potentially dangerous ventricular arrhythmias such as ventricular fibrillation.

US Patent 10,130,819 discusses a therapy approach in which the R-wave of the cardiac cycle is detected, generating a synchronization signal that triggers two events. First, an ablation therapy signal is generated a fixed period of time (50 milliseconds (ms)) after the synchronization signal. Second, a blanking period is set during which further synchronization signals are prevented from triggering further therapy. In some examples, US Patent 10,130,819 will then extend the blanking period if a further synchronization signal is received during the blanking period, to prevent noise or non-R-wave cardiac artifacts from erroneously triggering therapy. However, the patent does not discuss adjustments that will tailor the therapy to the patient's cardiac rhythm itself, instead addressing only the prevention of possible of erroneous therapy delivery by extending a blanking period.

Meanwhile, WO 2018/005511 A1 relates to the treatment of lung tissue by energy delivery, for example, to treat asthma, obstructive disorders, and other lung issues.

New and alternative systems to ensure safe operation are desired.

OVERVIEW

The invention is defined by the claims. In the following all methods of treatment are not claimed and are disclosed for illustrative purposes only.

The present inventors have recognized, among other things, that a problem to be solved is the need for new and/or alternative approaches to using the patients sensed cardiac signals to identify safe windows for application of ablation therapy. In some examples, the cardiac signal or ECG is detected and analyzed to determine an appropriate window for therapy delivery. While some prior systems may detect an R-wave and wait for expiration of a fixed interval to deliver therapy during an S-T segment, this approach fails to account for patient's individualized cardiac cycle timing and/or morphology, as well as failing to adapt to the patient's cardiac rate during execution of a therapy regimen. The present invention aims to solve this problem with a more adaptive approach. Some examples modify timing in response to cardiac rate. Some examples interrupt therapy delivery to sense the cardiac signal and update therapy window parameters. Some examples incorporate double checking of the sensed cardiac signal using noise detection, parameter matching, or other metrics, to confirm detection of the cardiac cycle and avoid incorrect therapy delivery timing.

A first illustrative and non-limiting example takes the form of an ablation system comprising: pulse generating means for generating output pulses for ablation purposes in response to one or more trigger signals; trigger means having an input means to receive cardiac signal information from a patient and issue trigger signals for issuance of ablation therapy; and a system controller coupled to at least each of the pulse generating means and the trigger means, the improvement comprising: the trigger means comprising analysis means to analyze the received cardiac signal information and identify safe periods of time for issuance of ablation therapy, and wherein the trigger means is configured to issue the trigger signals such that the pulse generating means generates output pulses for ablation purposes during such safe periods of time.

Additionally or alternatively, the trigger means is configured to operate the analysis means to analyze timing of the safe period after delivery of at least one ablation therapy within a therapy regimen.

The analysis means is configured to determine the safe period by calculating a cardiac beat rate for the patient and adjusting one or more of a start point for the safe period or a duration of the safe period.

Additionally or alternatively, the trigger means comprises sensing means for sensing cardiac cycles and identifying one or more of R-waves and T-waves therein, wherein the analysis means is configured to set the safe period by estimating a time after an R-wave at which a T-wave would be expected, and setting the safe period to start after the R-wave ends and end before the T-wave starts.

Additionally or alternatively, the trigger means comprises sensing means configured to sense a cardiac cycle and identify one or more features of the cardiac cycle including at least the R-wave; and the analysis means comprises determining means for determining a delay from R-wave onset to a safe start time in the cardiac cycle, and a period from the safe start time to an end time during which the safe period of time is defined.

Additionally or alternatively, the sensing means is configured to identify the T-wave as one of the features of the cardiac cycle; and the determining means is configured to identify the end time as a time prior to the T-wave.

Additionally or alternatively- the trigger means includes: sensing means configured to sense a cardiac signal and identify an event in the cardiac cycle; and confirmation means for confirming the identified event in the cardiac cycle: wherein the trigger means is configured to only issue trigger signals in response to identified, confirmed events.

Additionally or alternatively, die sensing means is configured to identify R-waves, and the confirmation means is configured to compare the identified event to an R-wave template to confirm that the identified event is an R-wave.

Additionally or alternatively, the sensing means is configured to identify R-waves, and the confirmation means is configured to calculate slew rate of the identified event and compare to a threshold to confirm that the identified event is an R-wave.

Additionally or alternatively, the system further includes a sensor for sensing one or more of sound, pressure or motion, wherein: the sensing means is configured to identify R-waves: and the confirmation means is configured to correlate an output of the sensor to the identified event to confirm the identified event is an R-wave.

Additionally or alternatively, the sensing means is configured to identify R-waves, and the confirmation means is configured to calculate a width of the identified event and comparing the width to a threshold or stored value to confirm that the identified event is an R-wave.

Additionally or alternatively, the sensing means is configured to identify R-waves, and the confirmation means is configured to compare an amplitude of the identified event to a stored amplitude of a previous R-wave to confirm that the identified event is an R-wave.

Additionally or alternatively, the confirmation means is configured to identified and count calculate one or more of turning points or inflection points in the cardiac signal associated with the identified event and compare to a threshold, and to reject as noise any identified event having turning points or inflection points higher than the threshold.

Additionally or alternatively, the trigger means is configured to operate across a series of cardiac cycles by: triggering ablation for one or more first cardiac cycles using first parameters defining the safe period; not triggering ablation for one or more second cardiac cycles occurring after the one or more first cardiac cycles and operating the analysis means to analyze the one or more second cardiac cycles and calculate second parameters defining the safe period; triggering ablation for one or more third cardiac cycles following the second cardiac cycles using the second parameters defining the safe period.

Additionally or alternatively, the system can further comprise a probe for delivering the output pulses from the pulse generator means to a patient.

A second illustrative, non-claimed and non-limiting example takes the form of a method of delivering an ablation therapy comprising: sensing one or more cardiac cycles of a patient: determining a safe period for delivering ablation therapy relative to an R-wave of the one or more cardiac cycles, the safe period defined at least by a delay interval relative to the R-wave: and using the safe period to deliver ablation therapy by sensing an R-wave of a subsequent cardiac cycle, waiting for expiration of the delay interval, and issuing the therapy.

Additionally or alternatively, the method further comprises re-analyzing the safe period after delivery of at least one ablation therapy within a therapy regimen.

Additionally or alternatively, the step of determining a safe period comprises calculating a cardiac rate of the patient using the sensed one or more cardiac cycles, and setting the safe period by estimating a time after the R-wave at which a T-wave would be expected, and setting the delay interval such that the safe period occurs before the T-wave.

Additionally or alternatively, the safe period defines both the delay interval and a duration, and the step of determining a safe period comprises calculating a cardiac rate of the patient using the sensed one or more cardiac cycles, and setting the safe period by estimating a time after the R-wave at which a T-wave would be expected, and setting the delay interval so that the safe period ends before the T-wave.

Additionally or alternatively, the step of determining a safe period comprises detecting, within a sensed cardiac cycle, each of an R-wave and a T-wave, and setting the delay interval such that the safe period will occur before the T-wave of a subsequent cardiac cycle.

Another illustrative, non-claimed and non-limiting example takes the form of a method of delivering an ablation therapy comprising: sensing a first cardiac cycle and generating a triggering signal in response to the sensed cardiac cycle; waiting for expiration of a calculated delay after the triggering signal, and delivering an ablation therapy signal to patient tissue; sensing one or more subsequent cardiac cycles and analyzing one or more features of the one or more subsequent cardiac cycles: adjusting the calculated delay in response to the analyzed one or more features of the one or more subsequent cardiac cycles; sensing a second cardiac cycle and generating a triggering signal in response to the sensed cardiac cycle; waiting for expiration of the adjusted calculated delay after the triggering signal, and delivering an ablation therapy signal to patient tissue.

Another illustrative, non-claimed and non-limiting example takes the form of a method of delivering an ablation therapy comprising: sensing one or more first cardiac cycles of a patient: analyzing the sensed one or more first cardiac cycles, identifying a safe window of time, relative to a detectable segment of the cardiac cycle, when ablation can be delivered safely, and setting one or more parameters to define the safe window relative to the detectable segment; delivering one or more ablation therapy signals by: detecting the detectable segment of at least one second cardiac cycle in a cardiac signal of the patient; using the set parameters to determine when the safe window will occur relative to the detected detectable segment; delivering at least one ablation therapy signal in the safe window: pausing to sense at least one third cardiac cycle, and adjusting the set parameters in response to the sensed at least one third cardiac cycle.

Additionally or alternatively, the method may further comprise sensing a fourth cardiac cycle and delivering an ablation therapy using the adjusted set parameters.

Still another illustrative, non-claimed and non-limiting example takes the form of a method of managing an ablation therapy, the ablation therapy being triggered by detection of cardiac cycles, the method comprising: using a first set of therapy delivery parameters, delivering one or more ablation therapy pulses, the first set of therapy delivery parameters comprising at least a delay interval, wherein the one or more ablation therapy pulses are delivered by sensing a cardiac signal component, waiting for expiration of the delay interval, and issuing at least one of the ablation therapy pulses; pausing therapy to sense one or more therapy-free cardiac cycles without interference from any ablation therapy pulses; analyzing the sensed one or more therapy-free cardiac cycles, and constructing a second set of therapy delivery parameters including an adjusted delay interval: and using the second set of therapy delivery parameters, delivering one or more ablation therapy pulses by sensing a cardiac signal component, waiting for expiration of the adjusted delay interval, and issuing at least one of the ablation therapy pulses.

Additionally or alternatively, the cardiac signal component is the cardiac R-wave.

Additionally or alternatively, the step of analyzing the one or more therapy-free cardiac cycles comprises determining timing information for at least the R-wave and T-wave of at least one of the therapy free cardiac cycles, and using the timing information to set the adjusted delay interval.

Another illustrative and non-limiting example takes the form of a method of managing an ablation therapy, the ablation therapy being triggered by detection of cardiac cycles, the method comprising: sensing an event in a cardiac cycle; comparing the sensed event to one or more parameters to confirm appropriate sensing of the event; and either: if the event is not appropriately sensed, withholding ablation therapy until at least a subsequent event is sensed; or if the event is appropriately sensed, delivering the ablation therapy.

Additionally or alternatively, the step of comparing the sensed event to one or more parameter comprises calculating a slew rate for a portion of the sensed event, and comparing the slew rate to one or more thresholds.

Additionally or alternatively, the step of comparing the sensed event to one or more parameters comprises performing a wavelet transformation of the sensed event to generate a set of basis functions and comparing at least one of the basis functions to an expected value.

Additionally or alternatively, the step of comparing the sensed event to one or more parameters comprises performing a correlation analysis relative to a stored template and determining if the sensed event matches the stored template.

Additionally or alternatively, the step of comparing the sensed event to one or more parameters comprises performing a correlation analysis relative to a previously sensed event and determining if the sensed event matches the previously sensed event.

Additionally or alternatively, the step of comparing the sensed event to one or more parameters comprises identifying a peak in an electrical signal and determining whether a peak in a sound, pressure, or motion signal correlates in time to the peak in the electrical signal.

Additionally or alternatively, the step of comparing the sensed event to one or more parameters comprises calculating a width associated with a peak in an electrical signal, and comparing the width to a threshold or stored value.

Additionally or alternatively, the step of comparing the sensed event to one or more parameters comprises calculating an amplitude of an electrical peak and comparing to a stored amplitude of a previous sensed event.

Additionally or alternatively, the step of comparing the sensed event to one or more parameters comprises counting turning points in a segment of the sensed event and comparing the quantity of turning points that are counted to one or more thresholds.

Additionally or alternatively, to any of the preceding examples and alternatives, the ablation therapy takes the form of a burst of individual pulses.

Another illustrative and non-limiting example takes the form of an ablation system comprising an ablation generator having one or more outputs for issuing ablation pulses, an ECG trigger circuit configured to analyze a cardiac ECG of a patient and generating trigger messages to trigger ablation pulse outputs, a pulse generator circuit for generating ablation pulse outputs, and a system controller coupled to at least each of the pulse generator circuit and the ECG trigger circuit, the system configured to perform a method as in any of the preceding examples and alternatives or additions thereto.

Additionally or alternatively, the system further comprises an ECG sensing system comprising at least one cutaneous electrode and an associated conductor, the ablation generator comprising at least one input for coupling to the at least one cutaneous electrode via the associated conductor, wherein the ECG trigger circuit comprises an analog filter, an amplifier, a digital filter, and an ECG analysis circuit adapted to identify heart beats.

This overview is intended to provide an introduction to the subject matter of the present patent application. It is not intended to provide an exclusive or exhaustive explanation of the invention. The detailed description is included to provide further information about the present patent application.

BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

Figure 1 shows an illustrative ablation system in use on a patient and target tissue;
Figure 2 shows an illustrative ablation system in block form;
Figure 3 shows an illustrative cardiac signal for reference;
Figures 4-5 are block process flow diagrams illustrating steps of select methods;
Figure 6 illustrates graphically a method of therapy delivery;
Figures 7-10 illustrate graphically ways of analyzing a cardiac signal to confirm beat detection;
Figure 11 is a block process flow diagram illustrating another method.

DETAILED DESCRIPTION

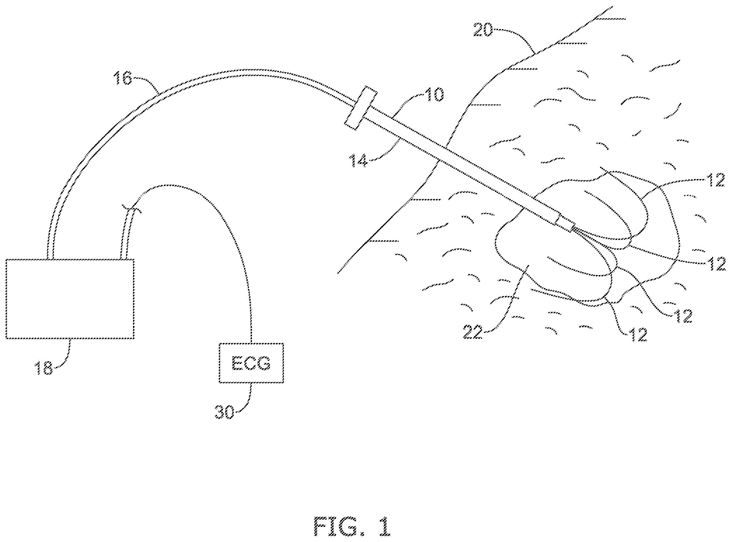

Figure 1 shows an illustrative ablation system in use on a patient and target tissue. An ablation probe 10 is shown and includes an elongated shaft 14 that extends to a plurality of tissue piercing electrodes 12 at a distal end thereof. The tissue piercing electrodes 12 can be extended or retracted once a target tissue 22 of a patient 20 is accessed. The proximal end of the apparatus is coupled by an electrical connection 16 to an ablation generator 18. Various features and potential enhancements of such a probe 100 are discussed in US Patents 5,855,576 and 6,638,277 as well as US PG Pat. Pub. No. 20190223943.

The system is shown having an ECG detector 30. In some examples, the ECG detector can be a separate unit having its own circuitry for sensing and analyzing a patient's ECG. When a separate unit is used, the ECG detector 30 may be configured to communicate by wired connectors or wireless link (such as Bluetooth or Medradio) to the ablation generator 18. In other examples, the ECG detector 30 may be a set of electrodes configured for placement in or on a patient's thorax to sense cardiac electrical signals, where the ablation generator 18 comprises internal circuitry for analyzing the received signals. In other examples, different or additional sensors may be provided, including accelerometers, microphones, optical detectors, etc. for sensing any of heart sounds, cardiac motion, and/or vascular or intracardiac flow or pressure signals. More than one sensing modality may be used separately or together (such as a heart sound sensor and a set of surface ECG electrodes), if desired.

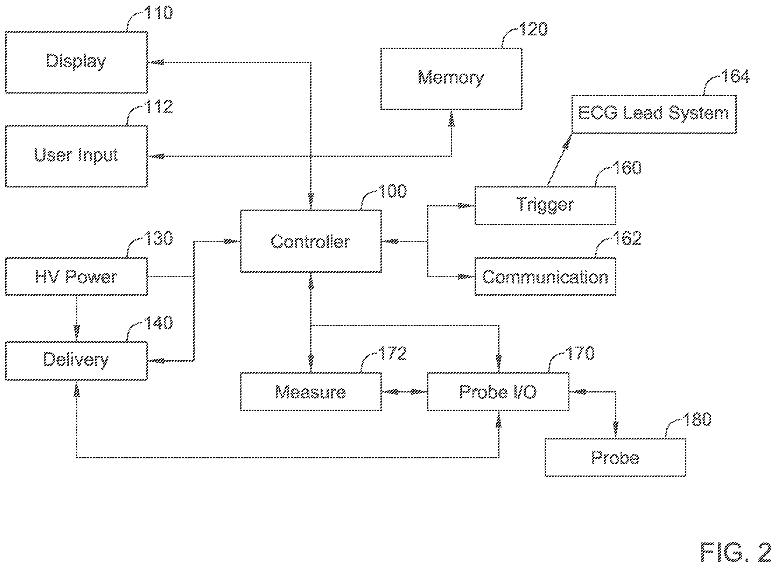

Figure 2 shows an illustrative ablation system in block form. The ablation system includes a controller 100 which may be, for example, a state machine, a microcontroller or microprocessor adapted to execute programmable instructions, which may be stored in a memory 120 that can also be used to store history, events, parameters, sensed conditions, alerts, and a wide variety of data such as template programs, information related to probes 180, and the like. The memory 120 may include both volatile and non-volatile memory types, and may include a port for coupling to a removeable memory element such as an SD card or thumb drive using a USB port.

The controller 100 is coupled to a display 110 and user input 112. The display 110 and user input 112 may be integrated with one another by including a touchscreen. The display 110 may be a computer screen and/or touchscreen and may also include lights and speakers to provide additional output statuses or commands, verbal prompts, etc. The user input 112 may include one or more of a keyboard, a mouse, a trackball, a touchpad, a microphone, a camera, etc. Any inputs by the user may be operated on by the controller 100. The controller 100 may include one or more application specific integrated circuits (ASICs) to provide additional functionality, such as an ASIC for filtering and analyzing an ECG for use as a trigger signal, or analog to digital conversion circuits for handling received signals from a probe apparatus.

For purposes herein, the controller 100 and/or the triggering circuit 160 may incorporate circuitry for receiving and processing an ECG-type signal. Such an ECG sensing system, whether in the controller 100 or triggering circuit 160 may be adapted for use in a system comprising at least one cutaneous electrode and an associated conductor. An input for coupling to the electrode and conductor can be provided on the ablation generator. The ECG processing circuitry may comprise one or more of an analog filter, an amplifier, a digital filter, and an ECG analysis circuit adapted to identify heart beats. For example, an analog filter may apply filtering to the incoming signal before or at its introduction to an analog-to-digital conversion circuit (which may also be provided), with the analog filter removing DC and high frequency components using, for example, a range of 1-4 Hz high pass filtering, and 40 or more Hz low pass filtering, such as by providing a 3 to 40 Hz bandpass. Digital filtering can use similar poles and zeros as desired, and may also include a bandstop filter for line signals in the 50 or 60 Hz range. Circuitry used in cardiac devices (pacemakers, defibrillators, recorders, and/or monitoring equipment whether implanted or external) can be used, for example. In some examples, the triggering circuit 160 may include its own microcontroller, state machine, and/or application specific integrated circuit, as well as associated memory and logic, to allow it to execute stored instruction sets to perform analysis, such as analysis of the received ECG to identify cardiac cycles, components of the cardiac cycle (R-wave, T-wave, and others as described herein), to perform beat/cycle detection, and/or to confirm the correctness or accuracy of beat detection using the methods for beat confirmation described below.

The controller 100 is also coupled to an HV Power block 130, which may comprise a capacitor stack or other power storage apparatus, coupled to a charger or voltage multiplier that provides a step up from standard wall power voltages to very high powers, in the hundreds to thousands of volts. A therapy delivery block 140 is shown as well and may include high power switches arranged in various ways to route high voltages or currents from the HV power 130 to a probe input/output (Probe I/O) 170, which in turn couples to a probe 180. In some examples, the HV power block 130 and Delivery block 140 may incorporate circuitry and methods described in US PG Pat. Pub. No. 20200289185, titled WAVEFORM GENERATOR AND CONTROL FOR SELECTIVE CELL ABLATION.

The Probe I/O 170 may include a smart probe interface that allows it to automatically identify the probe 180 using an optical reader interface (barcode or QR code) or using an RFID chip that can be read via an RF reader, or a microchip that can be read once the probe 180 is electrically coupled to a port on the Probe I/O 170. A measuring circuit 172 is coupled to the Probe I/O 170, and may be used to measure voltages, currents and/or impedances related to the probe, such as measuring the current flowing through a connection to the probe 180, or the voltage at an output of the Probe I/O 170. The Probe I/O 170 may comprise electrical couplings to the Probe 180 for purposes of therapy delivery, or for sensing/measurement of signals from the Probe 180, using for example sensing electrodes or sensing transducers (motion, sound, vibration, temperature or optical transducers, for example), as well as an optical I/O if desired to allow the output or receipt of optical energy, such as using optical interrogation of tissue or issuing light at therapeutic levels or even at ablation power levels. Not all of these options are required or included in some embodiments.

The controller 100 is also coupled to trigger circuitry 160 and/or communications circuitry 162. The trigger circuitry may include, for example, an ECG coupling port that is adapted to receive electrodes or an ECG lead system 164 for capturing a surface ECG or other signal from the patient for use in a triggered therapy mode. A communications circuit 162 may instead be used to wirelessly obtain a trigger signal, either a trigger that is generated externally, or a raw signal (such as an ECG) to be analyzed internally by the controller 100. The communication circuit 162 may include a transceiver having one or more of Bluetooth, Medradio or WIFI antennas and driver circuitry to wirelessly communicate status, data, commands, etc. before, during or after therapy regimens are performed. If desired, the trigger 160 may have a dedicated transceiver itself, rather than relying on the system communication block 160.

The probe 180 may take any suitable form, such as a LeVeen® needle, or a probe as shown in US Patent No. 5,855,576, 6,638,277, and/or US PG Pat. Pub. No. 2019/0223943, or other suitable ablation designs such as using multiple probes each comprising a needle electrode, either integrated into one structure or separately placed. The probe 180 may include one or more indifferent or return electrodes, such as plates that can be cutaneously placed.

In an example, the ablation generator 100 may have the capability to provide multiple ablation modes, such as by providing thermal ablation using first parameters, and non-thermal ablation, such as irreversible electroporation, using second parameters. The first and second parameters may result in different frequency content in the ablation output signal. The ablation generator may be configured to provide frequency content information for a programmed therapy output to the trigger circuit 160 for use by the ECG detector or detection circuit, in response to which the ECG detector or detection circuit may calculate values for use in analog or digital filtering to ensure removal of the ablation signal from the ECG waveform. For example, digital filtering may rely on filter coefficients tailored to particular frequencies: the filter coefficients may be adjustable in response to ablation signal content. In another example, an ECG detector or detection circuit may be configured with selectable filter settings to account for different frequency ranges used in different therapy modalities. In another example, the ECG detector or detection circuit may adapt analog filtering by, for example, modifying a capacitance or resistance (as by switching resistors or capacitors, by adding or removing capacitors or resistors from a circuit, or by adjusting a potentiometer value) used in a filter circuit to ensure removal of the ablation signal from the ECG waveform.

Ensuring removal of the ablation signal, in this context, may be performed by adjusting a filter characteristic frequency or corner value, or a filter Q value, for example. In a particular example, the ablation generator may be configured by the user to deliver a burst of therapy pulses, wherein the burst comprises pulse spikes separated by a spike interval. In a relatively simple approach, a value related to the spike interval may be provided. In a more complex example, the ablation generator may include a processor adapted to perform a frequency analysis, such as a fast Fourier transformation on a programmed therapy pattern, whether a burst or other signal is used. In other examples, the therapy output may be classified as high, medium, or low frequency depending on characteristics, and the ECG detector or detection circuit may be configured with corresponding filtering capabilities for each of the high, medium, 10 or low frequency categorizations. In another example, the ECG detector may use different filter or detection settings depending on whether a bipolar or unipolar therapy is being delivered. Some examples may instead omit this adaptive filtering capability if the expected frequency content of therapy outputs is, for example, above at least 500 Hz, since the typical frequency content of the cardiac signal is in a range of about 3 to about 40 Hz, meaning that low pass filtering methods that target the removal of line voltage signals (50 or 60 Hz, depending on geography) will also effectively block higher frequency content.

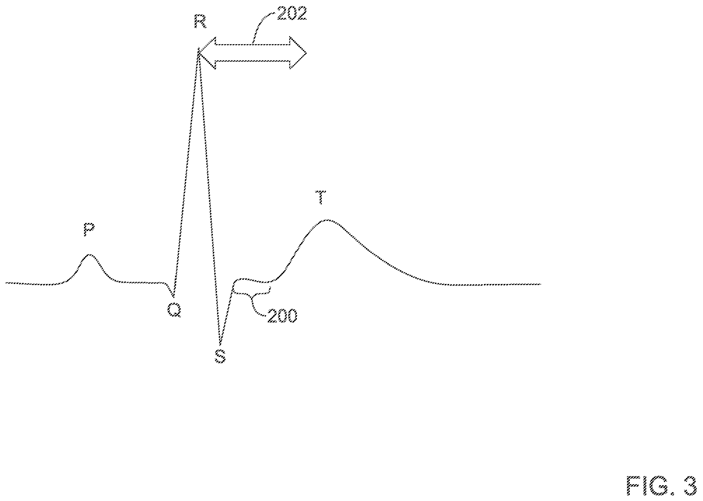

Figure 3 shows an illustrative cardiac signal for reference. A cardiac signal trace is shown using conventional naming techniques for several features of the cardiac signal, including a P-wave (atrial depolarization), the QRS complex (ventricular depolarization), in which the R-wave is shown as the largest peak, and the T-wave (ventricular repolarization). The heart is responsive to applied electrical stimulus depending on the timing of the stimulus relative to ECG waveform features. A stimulus applied on or before the P-wave can trigger a paced heartbeat, causing one ventricular contraction possibly out of step with the atrial contraction; a single such incident would not likely be harmful. A stimulus applied during the QRS complex and during a short period of time following the QRS complex, highlighted in the S-T interval at 200, will generally (absent very large amplitude) have no effect as the heart is refractory to stimulus during this time window. However, stimulus applied during the T-wave can be hazardous, as it may trigger ventricular fibrillation or a polymorphic ventricular tachyarrhythmia, either of which can cause the patient's blood pressure to drop to zero as the chaotic cardiac rhythm fails to pump blood effectively, leading within seconds to a loss of consciousness and within minutes to death, absent timely reversal.

Ablation systems are known to detect the R-wave or QRS complex and time therapy delivery to occur in the window 200. However, such systems typically do this by setting a fixed delay after the detection of the R-wave to start a safe window 200 for therapy delivery. The duration of the interval between the R-wave and the T-wave, highlighted at 202, is not fixed, and varies from patient to patient, as well as varying within a given patient in response to the beat rate. For example, various formulae (Bazett. Fridericia and Sagie formulae are examples) characterize the variation of the QT interval as a function of the interval between successive R-waves (the R-R interval). The RT interval would make up the vast majority of the QT interval. The Sagie formula is:

$$QT_{1c} = 1000 * (QT/1000 + 0.154*(1-RR))$$

Where $QT_{1c}$ is the expected QT interval at a given RR interval for a patient whose QT interval at 60 beats-per-minute (bpm), corresponding to a 1 second RR interval, is used in the formula as QT. Within a range of about 60 bpm to about 130 beats per minute, the QT interval, and likewise the RT interval, is reduced by about 20 milliseconds (ms) for every 10-bpm increase in heart rate What is a "normal" QT interval is the subject of some debate, but typically a normal QT interval at 60 bpm may be in the range of between about 330 to about 440 ms, with narrower bounds suggested by some researchers. In some examples, the QT interval is measured before or during a procedure and used to tailor therapy windows; in others, a "normal" QT interval, such as about 400 ms at 60 bpm, is assumed.

Also, patients vary in anatomy causing distinct cardiac signal conduction patterns, and patients having tumors and receiving drugs or other therapy for such tumors can display unusual conduction patterns. In order to set a fixed delay, adequate safety margin has to be built in to account for these variations, narrowing the usable time for therapy delivery beyond what may be necessary for any given patient.

In addition, in a real life, clinical application, a clean cardiac signal as shown in Figure 3 may not always occur. For example, in some patients, or in some lead configurations (that is, depending on how the ECG capturing electrodes are placed), the R-wave may be less prominent, or may be similar in amplitude to the T-wave, for example. Some patients have wider QRS complexes. Some patients will be subject to external noise, motion artifact, and/or myopotential noise (sometimes generated by skeletal muscle tissue or signals generated by the diaphragm). Some patients may experience aberrant beats, such as extrasystolic beats (premature ventricular contractions, for example). The ablation system, since it relies on the use of a safe window 200 to avoid harmful therapy outputs, should be designed to ensure accuracy of detection before setting the therapy delivery window 200.

The present inventors have noted these issues and seek to tailor the definition of the safe window 200 to the particular patient in some examples, to the cardiac rhythm in some examples, and to account for the potential for misidentifying the R-wave when triggering therapy.

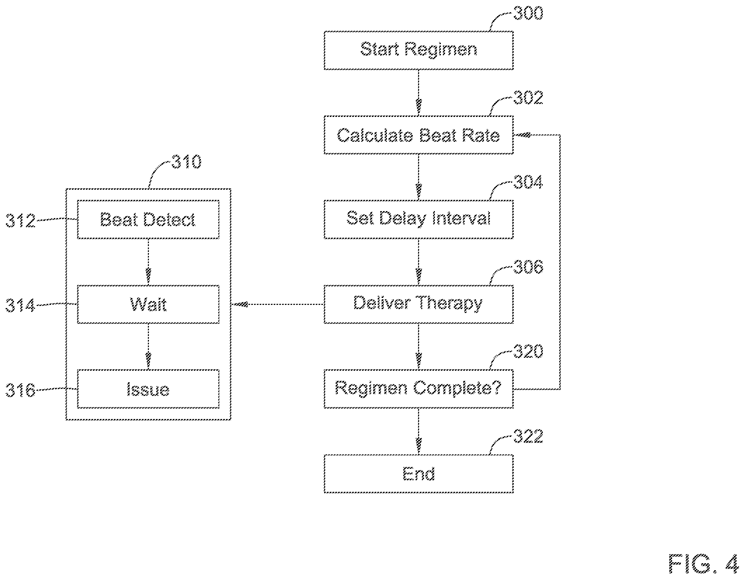

Figures 4-5 are block process flow diagrams illustrating steps of select methods. Starting with Figure 4, a method of ablation delivery is tailored to the patient's current cardiac state. The therapy regimen is started at 300. and the patient's cardiac beat rate is calculated at 302. The delay interval to use in therapy is then set, as indicated at 304, taking into account the cardiac rate.

For example, using a baseline QT interval of 400 ms, at 60 beats per minute, and a therapy window of 100 to 200 ms may then be defined, using a starting time that is about 50 to about 100 ms after the R-wave or QRS detection, and setting an end point that is about 50 to about 100 ms before the anticipated T-wave. In this assessment, the RT or QT interval may be used: generally speaking the RT interval will be shorter than the QT interval by anywhere from 20 to 40 milliseconds for most patients, though patients with wide QRS complexes may have still shorter RT intervals.

If the beat rate is measured as 100 beats per minute, the QT and/or RT intervals are then assumed to be shortened accordingly. One of the noted formulas above can be used (Bazett, Fridericia, or Sagie, for example), or a simplified version (20 millisecond reduction for each 10 bpm above 60) can be applied, and the therapy window timing and/or duration are calculated at 304. In some examples, a therapy window duration is fixed, while the delay interval before start of the therapy window may be adjusted: in others, both delay and duration are adjusted, in still other examples, only the therapy window duration is adjusted. Thus, the following chart reflects one illustrative example:

| Beat Rate | Assumed QT | Delay | Duration | Margin |
|-----------|-----------|-------|----------|--------|
| 60 bpm | 400 ms | 100 ms | 200 ms | 100 ms |
| 80 bpm | 360 ms | 80 ms | 180 ms | 100 ms |
| 100 bpm | 320 ms | 60 ms | 160 ms | 100 ms |
| 120 bpm | 280 ms | 50 ms | 130 ms | 100 ms |
| 140 bpm | 240 ms | 50 ms | 90 ms | 100 ms |

In this example, the delay before the start of a therapy window has a minimum value of 50 ms, and the margin from the end of the therapy window to the presumptive start of the T-wave is set at 100 ms, reflecting the fact that the front end delay poses less risk than if therapy occurs too close to T-wave onset. As noted, more sophisticated analysis to find an assumed QT may be used in other examples. The delay minimum and margin values shown in the table can be different in other examples. As can be seen from the chart, the window duration for therapy is over 100 milliseconds in several scenarios; this is far longer than is available with some prior art systems having a fixed window in which the only limit on therapy is if the patient's heart rate is observed to exceed a threshold. For prior systems, the therapy window is fixed in order to accommodate the highest acceptable heart rate, making for an unduly short window, and potentially extending the time spent in an ablation surgery. There are at least two ways the time spent in ablation surgery may be affected. First, to achieve successful therapy outcomes, one needs enough time to complete ablation of the target tissue. Second, for some therapy types, the cumulative effect of closely spaced-in-time pulses is used (see, for example, US Patent 8,926,606, which discusses achieving IRE with a pulse train of pulses that cannot achieve IRE individually but which, taken cumulatively, achieve IRE); the ability to extend the amount of time in a pulse train may enhance such therapy.

With the delay interval set at 304. the method proceeds with therapy delivery at 306. Therapy delivery 306 may include a subroutine shown at 310 including beat detection 312. followed by a waiting period 314, and then issuance of therapy output. The waiting period can be used to ensure that other analysis, such as confirmation of beat detection, can be completed prior to issuing a therapy output, in some examples. One or more therapy outputs across one or more heart beats can be generated at 306/310 before proceeding to block 320 to determine whether the therapy regimen is completed. If the regimen is incomplete, the method returns to block 302. When returning to block 302, any of several approaches can be taken. In one example, the return to block 302 occurs after a therapy output and before detection of a subsequent cardiac cycle, with rate calculated using the most recently detected R-waves or QRS complexes, to which therapy may have been applied. In other examples, the return to block 302 represents a therapy pause or interruption, and one or more cardiac cycles are observed to calculate the beat rate at 302 from cardiac cycles during which no ablation therapy is delivered. Such a pause is optional. After returning to block 302, blocks 304 and 306 are repeated, leading again to block 320. The process can iterate until the therapy regimen is completed, at which point the process ends at 322. Though not shown, when the beat rate is calculated, the method may compare the beat rate to a high or low boundary condition; if either boundary is violated, the therapy may be interrupted and an output alarm or other annunciation signal can be generated, indicating the cardiac rhythm, as sensed, is not within expected behavior. The physician can then determine whether therapy should proceed or cease, or if other action (defibrillation, bradycardia pacing, drug delivery, etc.) is needed.

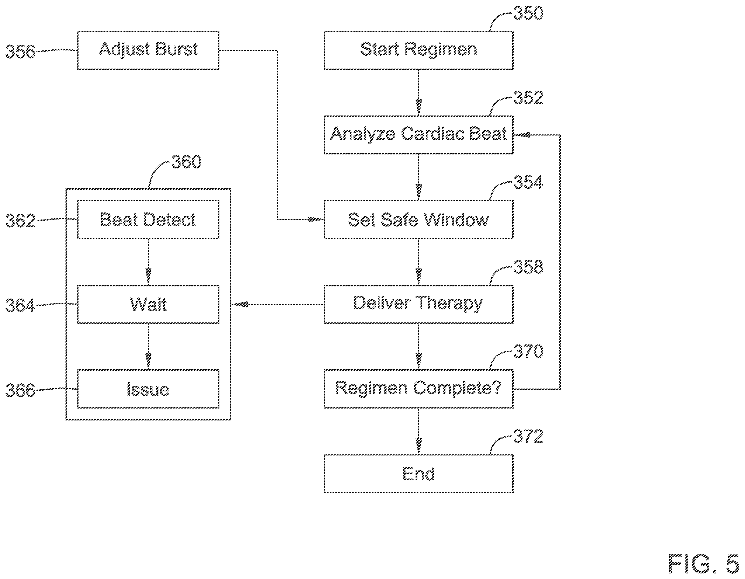

Figure 5 shows another example. Here, the therapy regimen starts at block 350, and a cardiac beat or cycle of the patient is analyzed at 352. The analysis in block 352 may be manual or automated, and may include identifying features of the cardiac signal such as, for example, the QT interval at a given cardiac rate. Other features can also be measured and/or recorded, such as QRS width, amplitude of any of the P, R, or T waves, shape of the P-wave or QRS complex, shape/elevation of the signal during the ST interval relative to baseline, the ratio of the R-wave to other features (P, Q, S. or T, for example), polarity of any of these waves, slew rate of any of the P-wave. QRS complex segments, or the T-wave, and/or the beat to beat stability of such features, for example.

In some examples, step 352 measures the current state without attempting to model cardiac rhythm behavior, such as by measuring the QT or RT interval. This data can then be fed directly to block 354 to set a safe window for therapy to be delivered following QRS or R-wave detection. As in other examples, the safe window may be set to start at some delay following the R-wave, and to terminate before the T-wave. Depending on the safe window duration, the burst to be delivered as ablation therapy can be adjusted, as noted at 356. For example, a therapy burst can be issued as a series of square wave pulses each having a pulse width, separated by an interpulse interval, up to a set quantity of the pulses. The duration of a burst is the product of the quantity of pulses times the pulse width plus interpulse interval, and this duration can be adjusted as indicated at 356 to fit within the safe window, or to maximize utilization of the safe window. Non-burst therapy can be delivered as well or instead.

In some examples, multiple bursts may be delivered in a single safe window. For example, some IRE and other non-thermal therapies can use very narrow pulse widths on the range of less than 10 microseconds; assuming an interpulse interval of 20 microseconds and pulse width of 5 microseconds, then a burst of 40 pulses can be delivered in 1 millisecond (25 microseconds times 40 pulses). Using an interburst period of 5 milliseconds, then 20 bursts could be delivered in a 120-millisecond therapy window, while only 15 bursts can be delivered in a 90-millisecond therapy window.

With the safe window set at 354, and, optionally, any burst parameters adjusted at 356, therapy is delivered as indicated at 358. Again, therapy delivery can include a subroutine shown at 360. with beat detection 362, a waiting time 364, and issuance of therapy at 366. The therapy delivery at 358 may operate subroutine 360 one or more times, as desired. If the regimen is complete at 370, the method ends: otherwise it returns to block 352.

An alternative example blends the methods of Figures 4-5 by using the method of Figure 5 in a first iteration, gathering cardiac beat data including the patient-specific QT interval information, and then using cardiac rate to make adjustments as shown in Figure 4 for one or more subsequent iterations. For example, the QT interval may be measured once a minute, while the beat rate is measured in an ongoing manner, with each used to tailor the therapy window for the patient and the patient's current state. In some examples, the method in Figure 5 performs beat analysis on beats/cardiac cycles that have not received therapy, and uses rate analysis without regard to whether a cardiac cycle receives therapy. A variety of blends of the two methods may thus be used.

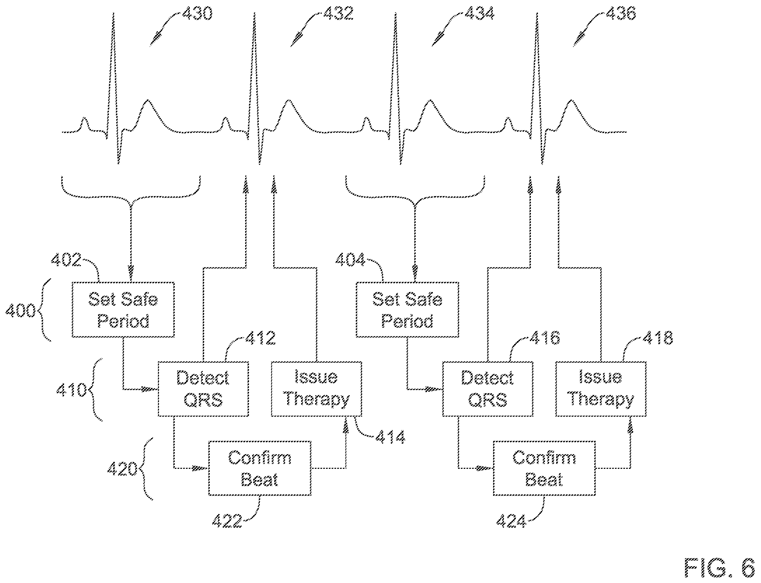

Figure 6 illustrates graphically a method of therapy delivery. Several enhancements and unique operational modes are highlighted together in Figure 6. First, as shown at 400 and 410, the safe period for ablation is repeatedly, for example, before each ablation therapy delivery. The approach shown delivers ablation therapy for alternating heart beats, and uses non-treated beats to determine therapy windows. For example, beat 430 is analyzed to set a safe period as indicated at 402, which is then used for delivery of therapy during a safe period of beat 432 by detecting the QRS complex at 412 and issuing therapy at 414. In addition, the method illustrated performs an additional analysis of beat confirmation at 422 prior to therapy being issued. Various methods of confirmation are discussed further below.

In the example, a safe period is determined at block 402 by analyzing beat 430. The analysis may, for example, identify the R-wave and T-wave in the beat 320, and then define parameters for the safe period for therapy delivery to start after the R-wave and end before the T-wave. The next beat 432 is sensed/detected at 412, with confirmation of the beat taking place at 422 before therapy is issued at 414 using the safe period settings from block 402. In a further example, the beat detection 412 and confirmation 422 are also performed for beat 430 to provide accurate data for analysis block 402. After therapy is issued at 414, the process repeats for beats 434, 436. with a safe period set at 404 (operating in this example similar to block 402) using beat 434. The beat at 436 is sensed/detected at 416, confirmed at 424, and then therapy is issued at 418.

In a further example, the detection of a QRS at 412 may come with identification of a fiducial point, such as the R-wave peak or an inflection point that precedes or follows the R-wave, for use in repeatedly starting the delay period. For example, if the sensing circuitry applies a detection threshold to the cardiac signal to detect the cardiac event, the point in time, relative to the actual Q or R wave where detection occurs may vary due to baseline drift or changes in signal morphology. By using the beat detection to next search for a fiducial point to use for delay and/or safe period calculation, the system may further tailor the analysis to the actual cardiac rhythm of the patient.

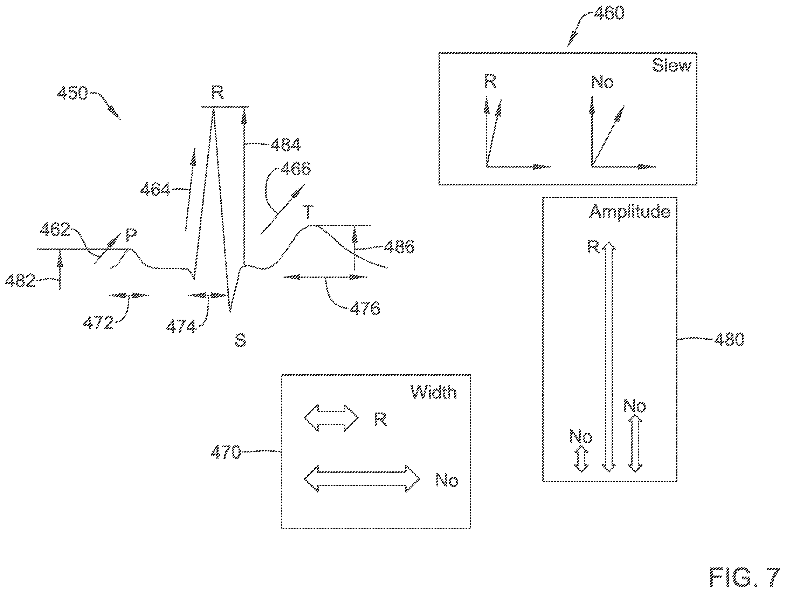

Figures 7-10 illustrate graphically ways of analyzing a cardiac signal to confirm beat detection. Starting in Figure 7, an idealized cardiac cycle is shown at 450 for reference. One illustrative way to confirm beat detection is by reference to slew rate, as shown at 460. Slew rate indicates how quickly voltage changes per unit time. In the example, the P-wave has a slew rate 462, the R-wave has a slew rate 464, and the T-wave has a slew rate 466. The R-wave slew rate 464 is characteristically greater than the slew rate for the other waveform features in this example. Thus a higher slew rate can be used to confirm the R-wave has been detected, while a lower slew rate is used to determine that the R-wave has not been detected. Where the QRS is detected, the longest positive slope period, or the first long slope period, or other characteristic, may be used to select from among the sloped segments of the QRS complex to use in a slew rate assessment. Parameters for distinguishing R-waves from T-waves and/or P-waves using slew rate, or other feature/-

characteristic, may be preset or may be calculated by analyzing one or more cardiac cycles of a patient.

Another illustrative way to confirm beat detection is by reference to width, as shown at 470. The P-wave has a width 472, the R-wave or QRS complex has a width 474, and the T-wave has a width 476. The T-wave width 476 is characteristically longer than either of the other two options. Thus, as shown at 470, width can be used to confirm detection of an R-wave, while a longer width (above a threshold of, for example, 100 milliseconds) can be used to flag an inappropriate T-wave detection. Some patients have wide QRS complexes, and the use of width may be tailored to such patients by measuring R-wave or QRS width in advance to set a threshold use in a width analysis. It may be noted that the P-wave and R-wave may not be distinguished readily using width in this example. That issue can be addressed, for example, by setting a sensitivity threshold to pass over the P-wave, which is typically much lower in amplified than either the R-wave or T-wave. In some examples, this may be a matter of configuring the QRS sensing electrodes if using, for example, surface electrodes, to place the electrodes in relatively lower positions relative to the heart, which will attenuate P-wave amplitude (which is generated in the atria) and thus detect, primarily, electrical signals generated in the ventricles.

Amplitude is another measure that can be used, as indicated at 480. The P-wave has a P-wave amplitude 482, the R-wave has an R-wave amplitude 484, and the T-wave has a T-wave amplitude 486. The R-wave amplitude 484 in this example is significantly higher than the other two 482, 486. Again, placement of sensing electrodes for the ECG detector or ECG detection circuit can be manipulated to aid in making this factor distinctive.

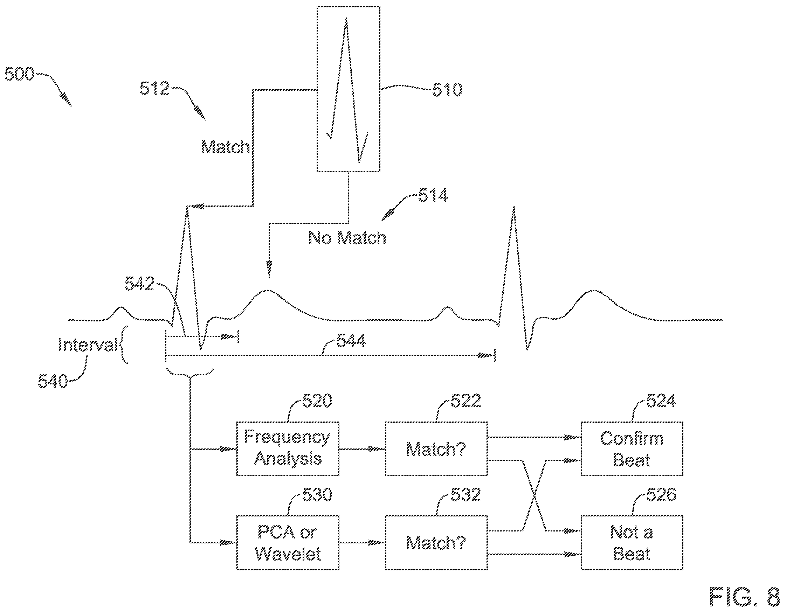

Figure 8 shows additional beat confirmation criteria. A correlation analysis, such as correlation waveform analysis (CWA), can be used by reference to a template 510 of an R-wave or QRS complex. The template 510 may be a stored or static template formed at a given point in time and used to analyze a plurality of detected events, or may be a continuously updated or dynamic template, such as being taken from an immediately preceding confirmed beat. When the template is matched, as indicated at 512, the detected event can be confirmed as a QRS complex or R-wave. When the template does not match, as would be the case with a T-wave detection compared to an R-wave template, no match is found and the detected event is not treated as a QRS complex.

The detection may be analyzed using frequency analysis as indicated at 520. For example, R-waves are known to have characteristic frequencies that are typically between about 20-40 Hz, while T-waves have characteristic frequencies of about 10 Hz and lower Thus frequency analysis 520 can be used to see whether a detected event matches an expected frequency range, which can be pre-set or may be based on analysis of a patient's own cardiac cycle. At 522 the method determines whether a sensed/detected event matches the expected frequency range: if so, the beat is confirmed at 524 and, if not, the beat is not confirmed as indicated at 526. The P-wave frequency content tends to overlap that of the R-wave, so other features may be used to avoid P-wave detection or to distinguish P-waves from R-waves or QRS complexes, as discussed above.

A transform analysis may be used instead. For example, a Principle Components Analysis (PCA) or Wavelet transform may be used. Each process generates a set of characteristic eigenvectors of the signal under analysis, and the different components of the cardiac cycle, when analyzed, will have different wavelet or PCA characteristic features, allowing R-waves to be distinguished from other features. At 530 a transform is performed, and at 532 the method determines whether the output of the transform matches expected values or not. The expected values in block 532 may be pre-set, or may be generated by analyzing one or more cardiac cycles of the patient using at least QRS components and, potentially, other components, to determine differences between the transform solutions of different components of the cardiac cycle. When a match to expected values for an R-wave or QRS complex occurs, the beat is confirmed at 524 and, if not, the beat is not confirmed at 526.

Still another method is to review intervals between detections, as indicated at 540. The interval from one R-wave to the next is generally pretty stable absent cardiac arrhythmia. A sudden change in R-R or QRS-QRS intervals may indicate onset of arrhythmia, while inconsistency in R-R intervals over time may indicate a conducted atrial arrhythmia. Interval analysis can thus be used by assuming that, absent misdetection and/or arrhythmia, the R-R or QRS-QRS interval is generally stable. Thus, a short interval 542 between an R-wave detection and a T-wave detection can be readily distinguished from a long interval 544 between two R-waves, for example. For the interval analysis, a preceding R-R interval may be stored, or an ongoing average R-R interval (such as the average of four preceding R-R intervals) may be maintained. Again, when there is a match, the beat can be confirmed, and mismatch will not result in the beat being confirmed.

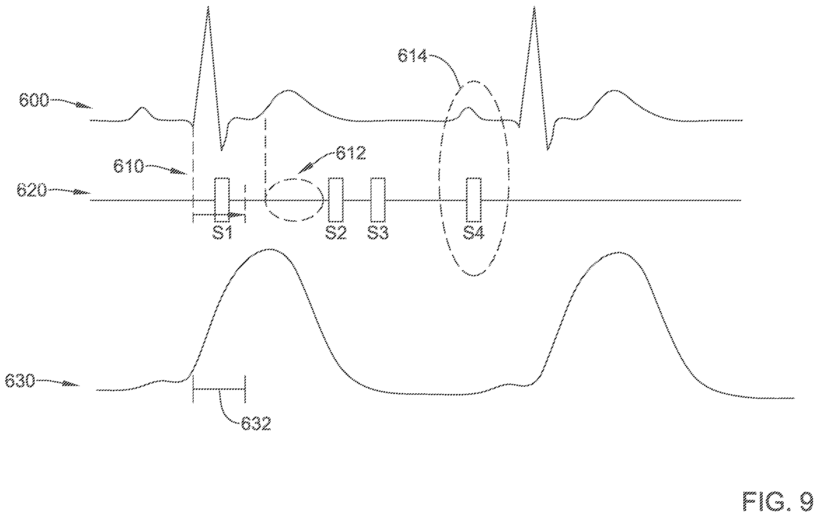

Figure 9 shows another example. Here the electrical cardiac signal is shown as ECG 600, with heart sound signals shown at 620 and pressure signals shown at 630. Referring first to the combination of heart sounds with the ECG. when an R-wave occurs the first heart sound S1, caused by closing of the tricuspid and mitral valves as the ventricles depolarize and contract, will appear shortly thereafter, typically in less than 100 milliseconds. The system may look for the occurrence of S1 to confirm the detection of the R-wave as shown at 610. The T-wave, on the other hand, is not correlated in time with a heart sound in the same fashion as the R-wave, as shown at 612. The second heart sound, S2, is caused by closure of the aortic and pulmonary valves in response to pressure dropping in the ventricle below that of the aorta, and occurs well after the T-wave onset. The third and fourth heart sounds S3 and S4 can be readily distinguished by their much lower amplitude, and so the fact that the fourth heart sound may correlate in time to the P-wave is generally a non-issue (assuming the fourth heart sound is even present). In use, the delay from R-wave detection to the safe window may be set to ensure that the first heart sound can be observed before ablation therapy delivery. An implantable or wearable transducer may be used to observe heart sounds.

The pressure signal shown at 630 may also be used. As shown at 632, the pressure signal shows a significant rise associated with ventricular depolarization as the ventricle contracts. No such rise is correlated with any other component of the cardiac cycle, and so, again, this feature may be used. An implantable or wearable transducer may be used to observe the pressure signal. An analog for the pressure signal can be captured by pulse oximetry, though the use of extremity-placed pulse oximetry may limit the value of this marker if the pulse flow signal is delayed by virtue of the distal placement, relative to the heart, of the sensor.

The failure to confirm a beat can be used in any of these examples to inhibit therapy delivery. The system may record such a failure and then resumes sensing to identify a subsequent possible cardiac cycle, and will again analyze the subsequent cycle to confirm beat detection. If several detections consecutively fail to be confirmed, the system may generate an error and alert the user.

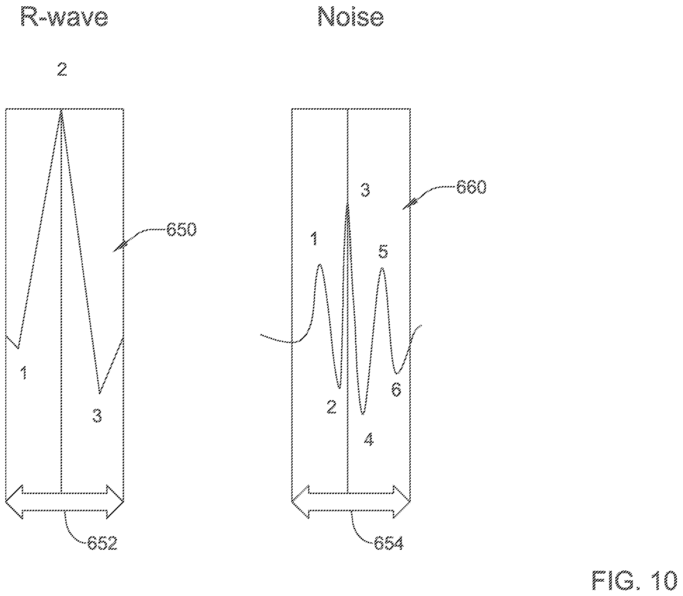

Figure 10 shows a method of using turning points to distinguish detection of a cardiac originating signal from a non-cardiac noise source. An R-wave morphology is shown at 650, and a noise signal is shown at 660. Such noise may be non-biological, such as noise generated by a device worn by the patient or in proximity to the patient during the ablation surgery, or may come from a myopotential such as skeletal muscle noise or diaphragm-related signals. These non-cardiac signals will tend to have higher frequency content than the cardiac signals, and so when analyzing within a window, as indicated at 652, and 654, the number of turning points in the window for each signal will be different. The window duration may be, for example, anywhere from 50 to 100 milliseconds, for example, and may be tailored to the particular patient by determining turning points 1 and 3 of the R-wave or QRS complex signal 650, which may correspond to the start of the longest rise period in the R-wave and the end of the longest fall period in the R-wave, as shown. The noise signal 660 is shown as having more turning points (6 here) than the R-wave (3 here), and this difference may be used in some examples to distinguish the noise signal 660 from the R-wave signal 650. For example, when analyzing the turning points, a threshold can be set, such as 5 turning points. A signal having more than the threshold number of turning points can be rejected as noise, and the beat caused by the noise will not be confirmed. In some examples, the turning points can be determined by the use of an ongoing calculation of the first derivative of the incoming signal, with first derivative zeros being counted. In another example, inflection points (second derivative zeros) can be used instead of turning points.

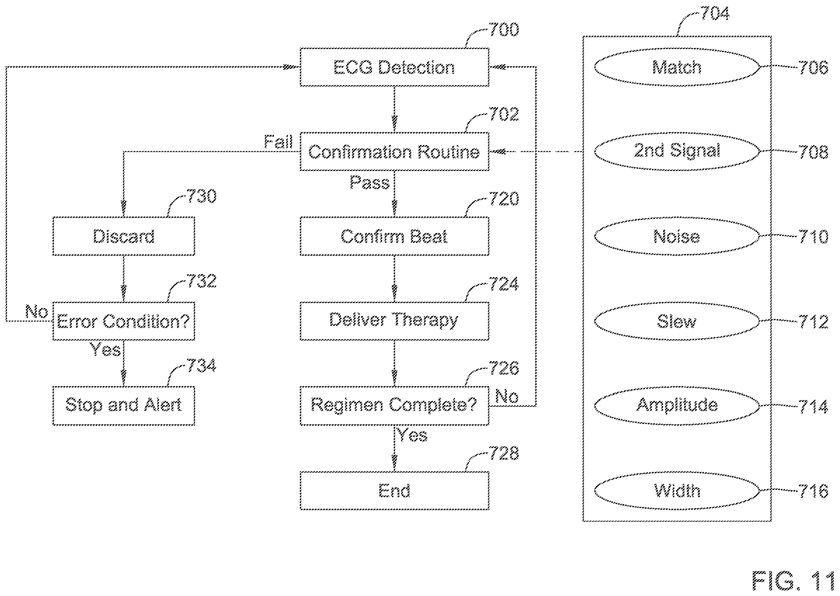

Figure 11 is a block process flow diagram showing the use of the various confirmation processes of Figures 7-10. Any suitable number of features can be used in combination to confirm, or to prevent confirmation of, beats. The method flow begins at block 700 where ECG detection is performed. In ECG detection, an event, such as a slope or peak, in the ECG is detected and treated as a detected event. For example, the ECG detection block 700 can be performed by electronically comparing an ECG signal amplitude (rectified or not) to a sensing threshold, which may be fixed or time varying (such as a time decaying sensing threshold). In some examples a detection threshold can be applied at block 700 that decays or drops over time from a first, relatively high amplitude, to a later, second, relatively lower amplitude, with the first amplitude chosen so as to exceed an expected amplitude of the patient's T-wave, and the second amplitude chosen to exceed an expected amplitude of the patient's P-wave. The setting of the first and second amplitude may be performed from a stored value or values, or may be tailored to a particular patient by analysis of one or more cardiac cycles. A refractory period may be applied at least during the period of therapy output to prevent detection of the therapy itself, and, if desired, for some period thereafter to avoid detection of the T-wave, if desired. US. Patent No. 8,565,878 discusses some illustrative devices and methods for detecting cardiac cycles.

The detected events in the ECG signal may then be passed to a confirmation routine, as indicated at 702. The confirmation routine may be use one or more of the analysis listed at 704. For example, matching of a signal associated with a detected event in the ECG signal to a template (whether static or dynamic, or in a CWA, Wavelet, PCA, frequency, or other morphology analysis) can be used as indicated at 706. US Patent Application Publication Numbers 20090259271, 20100004713, and 20110098585, for example, discuss identifying over-detected cardiac events prior to confirmation. Confirmation using a second signal, such as sound, motion, optical (oximetry) or pressure signal may be performed as indicated at 708 by, for example, seeking to correlate an ECG detected event with a second signal. Confirmation may include analyzing a detection to see if it is caused by or contaminated with noise, as indicated at 710, such as by use of an analysis as in US Patent No. 7,248,921. Confirmation may also refer to features of the signal itself, such as slew rate 712, amplitude 714, and/or width 716. Each feature in block 704 has been explained in various examples provided above. Any number of the analysis in block 704, from one to all, may be used.

If the detected event passes the confirmation routine, the beat is confirmed at 720, and therapy is delivered at 724. Next the method determines whether the therapy regimen is completed, at 726 and, if so, the method ends at 728. If the regimen is not yet completed, the method returns to block 700.

If the detected event fails the confirmation routine, the method goes to block 730. The ECG detection is discarded at 730 to prevent therapy delivery based on the non-confirmed detection. The method can then determine whether an error condition exists. For example, if no beats have been confirmed for a period of time (3-5 seconds, or less or more, for example), this may be considered an error - either the patient is experiencing asystole or an arrhythmia that has interrupted the ECG signal, or something has occurred to cease ECG signal sensing, such as disconnection of a lead or electrode, interruption of communication with an ECG detector, or a lead/electrode becoming displaced relative to the patient tissue/heart. In another example, if several detections in a row fail confirmation, or if a threshold quantity of ECG detections fail confirmation within a time period (such as 3 failures within 5 seconds, or some other combination), this may indicate any of a variety of issues including potential arrhythmia onset, displacement or disconnection of electrodes, introduction of a noise source impairing sensing, etc. If an error condition is found at 732, the system stops/interrupts the therapy routine and generates an alert to the physician, as shown at 734. An alert may by any one or any combination of audible, visual and/or tactile alerts. If no error condition is found at 732, the method returns to block 700.

In Figure 11, though not shown, the parameters of the therapy delivery at 724 can be adjusted using the methods illustrated above in Figures 4-6, such as tailoring the safe period for delivery of therapy according to features of the detected cardiac signal including, for example and without limitation, cardiac rate and intervals between features of the cardiac cycle. The therapy regimen may be interrupted, such as shown in Figure 6, to allow safe periods for delivery of therapy to be adjusted.

Any of the above examples can be further tailored for the context of cardiac pacing. In an example, a patient may receive cardiac pacing therapy during an ablation therapy, either as an adjunct to the ablation therapy or because the patient is dependent on the pacemaker. The pacemaker may take any form (implanted transvenous or leadless pacemaker, for example, or an external pacemaker), and may either communicate with or be detectable by die ablation system. In an example, pace outputs can be identified by the ablation system sensing for such pulses (which typically have a distinct frequency characteristic and/or shape as compared to cardiac signals) or sensing for pace captured beats (which are morphologically distinct from native beats). The ablation therapy can be adapted to the pacing rate. Moreover, in some examples, the cardiac rate can be overdriven (intentionally paced at higher than intrinsic rate), which can be useful to ensure a predictable cardiac cycle or to increase the quantity of ablation outputs that can be generated over time. For example, if the ablation output burst is delivered once per cardiac cycle, overdrive pacing the heart, within safe physiological boundaries, would increase the number of cardiac cycles per unit time, potentially reducing the time needed to complete a procedure. In another example, an increased pace rate can be used to reduce the recovery time between ablation outputs, potentially increasing thermal effects (if needed), or preventing cellular wall recovery to amplify the effect of electroporation. In an example, electroporation can be delivered as the paced cardiac rate is kept relatively higher, until a thermal effect is observed, at which point pacing output can be reduced.

In an illustrative, non-limiting example, an ablation system comprises pulse generating means for generating output pulses for ablation purposes in response to one or more trigger signals (such pulse generating means includes an HV Power block 130 and a delivery block 140, as shown and described above), trigger means having an input means to receive cardiac signal information from a patient and issue trigger signals for issuance of ablation therapy (such trigger means may be as in block 160. and/or may include executable instructions stored in memory 120 for execution by controller 100, each as shown and described above); and a system controller coupled to at least each of the pulse generating means and the trigger means (such as controller 100 as shown and described above). In the example, an improvement may comprise the trigger means comprising analysis means to analyze the received cardiac signal information and identify safe periods of time for issuance of ablation therapy (operation of such an analysis means, which may be implemented as a controller executable instruction or in, for example, a dedicated circuit such as an application specific integrated circuit is shown and described in examples in each of Figures 4-6, including in Figure 4 the calculation of beat rate 302 and setting of the delay interval 304. as well as in Figure 5 analysis at 352 of the cardiac beat and setting of the safe window at 354, and again in Figure 6 at 400, including blocks 402 and 404 which analyze a cardiac beat to determine when the safe period occurs for a subsequent beat), and wherein the trigger means is configured to issue the trigger signals such that the pulse generating means generates output pulses for ablation purposes during such safe periods of time (this utilization of the trigger means is described relative to blocks 160, 100 and 130/140 of Figure 2, and illustrated in method examples of Figure 4 at 310, Figure 5 at 360. and Figure 6 at each of 412/422/414 and 416/424/418).

Additionally or alternatively, the trigger means is configured to operate the analysis means to analyze timing of the safe period after delivery of at least one ablation therapy within a therapy regimen (Figure 4 shows a method that iterates from 320 back to 302. where timing would be reanalyzed. Figure 5 shows a method that iterates from block 370 to 352, where timing and the safe window/period would be reanalyzed, and Figure 6 shows analysis of beat 434 after therapy is issued on beat 432).

Additionally or alternatively, the analysis means is configured to determine the safe period by calculating a cardiac beat rate for the patient and adjusting one or more of a start point for the safe period or a duration of the safe period (Figure 4 shows expressly the adjustment of starting point, and associated text indicates also adjustment of the duration of the safe period, with blocks 302/304).

Additionally or alternatively, the trigger means comprises sensing means for sensing cardiac cycles and identifying one or more of R-waves and T-waves therein, wherein the analysis means is configured to set the safe period by estimating a time after an R-wave at which a T-wave would be expected, and setting the safe period to start after the R-wave ends and end before the T-wave starts (Figure 4 shows expressly the adjustment of starting point, and associated text indicates also adjustment of the duration of the safe period, with blocks 302/304, and performing this step specifically with R-waves and T-waves considered is described in text associated with at least Figure 4).

Additionally or alternatively, the trigger means comprises sensing means configured to sense a cardiac cycle and identify one or more features of the cardiac cycle including at least the R-wave (such a sensing means is described as including, for example, digital and analog filtering capability as well as amplification and analog-to-digital conversion capability in various examples above, as components of one or the other of a controller 100 or trigger block 160); and the analysis means comprises determining means for determining a delay from R-wave onset to a safe start time in the cardiac cycle, and a period from the safe start time to an end time during which the safe period of time is defined (this approach to analysis is shown in Figure 5, block 352and associated text, and also in Figure 6, blocks 402, 404, and associated text with analysis of the R-wave and T-wave features of beats to set safe periods).

Additionally or alternatively, the sensing means is configured to identify the T-wave as one of the features of the cardiac cycle; and the determining means is configured to identify the end time as a time prior to the T-wave (this approach to analysis is shown in Figure 5, block 352 and associated text, and also in Figure 6, blocks 402, 404, and associated text with analysis of the R-wave and T-wave features of beats to set safe periods).

Additionally or alternatively, the trigger means includes: sensing means configured to sense a cardiac signal and identify an event in the cardiac cycle (such a sensing means is described as including, for example, digital and analog filtering capability as well as amplification and analog-to-digital conversion capability in various examples above, as components of one or the other of a controller 100 or trigger block 160); and confirmation means for confirming the identified event in the cardiac cycle; wherein the trigger means is configured to only issue trigger signals in response to identified, confirmed events (such confirmation means can include, for example, a microcontroller, state machine, and/or ASIC, and associated memory and circuitry, for performing the beat confirmation methods described relative to blocks 422, 424 in Figure 6 and/or block 702 in Figure 11).

Additionally or alternatively, the sensing means is configured to identify R-waves (such a sensing means is described as including, for example, digital and analog filtering capability as well as amplification and analog-to-digital conversion capability in various examples above, as components of one or the other of a controller 100 or trigger block 160), and the confirmation means is configured to compare the identified event to an R-wave template to confirm that the identified event is an R-wave (as noted in Figure 11, at 706, using any of the methods shown in Figure 8, for example).

Additionally or alternatively, the sensing means is configured to identify R-waves (such a sensing means is described as including, for example, digital and analog filtering capability as well as amplification and analog-to-digital conversion capability in various examples above, as components of one or the other of a controller 100 or trigger block 160), and the confirmation means is configured to calculate slew rate of the identified event and compare to a threshold to confirm that the identified event is an R-wave (as noted in Figure 11, at 712 and shown in Figure 7 at 460).

Additionally or alternatively, the system may further comprise a sensor for sensing one or more of sound, pressure, or motion (as noted relative to the provision of an external ECG sensor which may instead be, or may additionally have, such sensors, and/or wherein such sensors can be provided in or on a probe 180 as shown and described above), wherein: the sensing means is configured to identify R-waves; and the confirmation means is configured to correlate an output of the sensor to the identified event to confirm the identified event is an R-wave (as shown in several examples of Figure 9 and noted in Figure 11 at block 708).

Additionally or alternatively, the sensing means is configured to identify R-waves, and the confirmation means is configured to calculate a width of the identified event and comparing the width to a threshold or stored value to confirm that the identified event is an R-wave (at 716 of Figure 11 and shown in Figure 7 at 470).

Additionally or alternatively, the sensing means is configured to identify R-waves, and the confirmation means is configured to compare an amplitude of the identified event to a stored amplitude of a previous R-wave to confirm that the identified event is an R-wave (as noted at 714 of Figure 11 and in Figure 7, block 480).

Additionally or alternatively, the confirmation means is configured to identify and count one or more of turning points or inflection points in the cardiac signal associated with the identified event and compare to a threshold, and to reject as noise any identified event having turning points or inflection points higher than the threshold (as noted at 710 in Figure 11 and illustrated in Figure 10 and associated text).

Additionally or alternatively the trigger means is configured to operate across a series of cardiac cycles by: triggering ablation for one or more first cardiac cycles using first parameters defining the safe period; not triggering ablation for one or more second cardiac cycles occurring after the one or more first cardiac cycles and operating the analysis means to analyze the one or more second cardiac cycles and calculate second parameters defining the safe period, triggering ablation for one or more third cardiac cycles following the second cardiac cycles using the second parameters defining the safe period (this sequence is illustrated in Figure 6 with treatment for the cardiac cycle at 432, recalculation of the safe period at 434, and use of the recalculated parameters for issuing therapy during cardiac cycle 436).

Additionally or alternatively, the system may include a probe for delivering the output pulses from the pulse generator means to a patient (a probe 10 is shown in Figure 1, and again at 180 in Figure 2).

Each of these non-limiting examples can stand on its own, or can be combined in various permutations or combinations with one or more of the other examples.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic or optical disks, magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description.

The scope of the invention should be determined with reference to the appended claims.

**Claims**

1. An ablation system (18) comprising:

   pulse generating means (130, 140) for generating output pulses for ablation purposes in response to one or more trigger signals;
   trigger means (160) having an input means (164) to receive cardiac signal information from a patient and issue trigger signals for issuance of ablation therapy; and
   a system controller (100) coupled to at least each of the pulse generating means and the trigger means, wherein the trigger means comprises analysis means to analyze the received cardiac signal information and identify safe periods of time for issuance of ablation therapy, and wherein the trigger means is configured to issue the trigger signals such that the pulse generating means generates output pulses for ablation purposes during such safe periods of time, **characterised in that**
   the analysis means is configured to determine the safe period by calculating a cardiac beat rate for the patient and adjusting one or more of a start point for the safe period or a duration of the safe period based on the calculated cardiac beat rate.

2. The ablation system of claim 1 wherein the trigger means is configured to operate the analysis means to analyze timing of the safe period after delivery of at least one ablation therapy within a therapy regimen.

3. The ablation system of claim 1 wherein the trigger means comprises sensing means for sensing cardiac cycles and identifying one or more of R-waves and T-waves therein, wherein the analysis means is configured to set the safe period by estimating a time after an R-wave at which a T-wave would be expected, and setting the safe period to start after the R-wave ends and end before the T-wave starts.

4. The ablation system of claim 1 wherein:

   the trigger means comprises sensing means configured to sense a cardiac cycle and identify one or more features of the cardiac cycle including at least the R-wave; and

the analysis means comprises determining means for determining a delay from R-wave onset to a safe start time in the cardiac cycle, and a period from the safe start time to an end time during which the safe period of time is defined.

5. The ablation system of claim 4 wherein the sensing means is configured to identify the T-wave as one of the features of the cardiac cycle; and the determining means is configured to identify the end time as a time prior to the T-wave.

6. The ablation system of claim 1 wherein the trigger means includes:

sensing means configured to sense a cardiac signal and identify an event in the cardiac cycle; and confirmation means for confirming the identified event in the cardiac cycle; wherein the trigger means is configured to only issue trigger signals in response to identified, confirmed events.

7. The ablation system of claim 6 wherein the sensing means is configured to identify R-waves, and the confirmation means is configured to compare the identified event to an R-wave template to confirm that the identified event is an R-wave.

8. The ablation system of claim 6 wherein the sensing means is configured to identify R-waves, and the confirmation means is configured to calculate slew rate of the identified event and compare to a threshold to confirm that the identified event is an R-wave.

9. The ablation system of claim 6 further comprising a sensor for sensing one or more of sound, pressure or motion, wherein:

the sensing means is configured to identify R-waves; and the confirmation means is configured to correlate an output of the sensor to the identified event to confirm the identified event is an R-wave.

10. The ablation system of claim 6 wherein the sensing means is configured to identify R-waves, and the confirmation means is configured to calculate a width of the identified event and comparing the width to a threshold or stored value to confirm that the identified event is an R-wave.

11. The ablation system of claim 6 wherein the sensing means is configured to identify R-waves, and the confirmation means is configured to compare an amplitude of the identified event to a stored amplitude of a previous R-wave to confirm that the identified event is an R-wave.

12. The ablation system of claim 6 wherein the confirmation means is configured to identify and count one or more of turning points or inflection points in the cardiac signal associated with the identified event and compare to a threshold, and to reject as noise any identified event having turning points or inflection points higher than the threshold.

13. The ablation system according to any preceding claim wherein the trigger means is configured to operate across a series of cardiac cycles by:

triggering ablation for one or more first cardiac cycles using first parameters defining the safe period; not triggering ablation for one or more second cardiac cycles occurring after the one or more first cardiac cycles and operating the analysis means to analyze the one or more second cardiac cycles and calculate second parameters defining the safe period, triggering ablation for one or more third cardiac cycles following the second cardiac cycles using the second parameters defining the safe period.

14. The ablation system according to any of claims 1-13 further comprising a probe for delivering the output pulses from the pulse generator means to a patient.

**Patentansprüche**

1. Ablationssystem (18) aufweisend:

eine Pulserzeugungseinrichtung (130, 140) zum Erzeugen von Ausgabepulsen für Ablationszwecke als Antwort auf ein oder mehrere Auslösesignale;

eine Auslöseeinrichtung (160) mit einer Eingabeeinrichtung (164), um Kardiosignalinformation von einem Patienten zu empfangen und Auslösesignale zur Ausgabe von Ablationstherapie auszugeben; und

eine Systemsteuerung (100), die zumindest sowohl mit der Pulserzeugungseinrichtung als auch mit der Auslöseeinrichtung gekoppelt ist, wobei

die Auslöseeinrichtung eine Analyseeinrichtung aufweist, um die empfangene Kardiosignalinformation zu analysieren und sichere Zeitintervalle für die Ausgabe von Ablationstherapie zu identifizieren, und wobei die Auslöseeinrichtung konfiguriert ist, die Auslösesignale derart auszugeben, dass die Pulserzeugungseinrichtung Ausgabepulse für Ablationszwecke während solcher sicherer Zeitintervalle erzeugt,

**dadurch gekennzeichnet, dass**

die Analyseeinrichtung konfiguriert ist, das sichere Intervall zu bestimmen durch Berechnen einer Herzschlagrate für den Patienten und Anpassen eines Startpunkts für das sichere Intervall und/oder Anpassen einer Dauer des sicheren Intervalls auf Basis der berechneten Herzschlagrate.

2. Ablationssystem nach Anspruch 1, wobei die Auslöseeinrichtung konfiguriert ist, die Analyseeinrichtung zu betreiben, um Terminierung des sicheren Intervalls zu analysieren nach Abgabe mindestens einer Ablationstherapie in einem Therapieregime.

3. Ablationssystem nach Anspruch 1, wobei die Auslöseeinrichtung aufweist: eine Detektionseinrichtung zum Detektieren von Kardiozyklen und zum Identifizieren einer oder mehrerer R-Wellen und/oder T-Wellen darin, wobei die Analyseeinrichtung konfiguriert ist, das sichere Intervall zu setzen durch Schätzen eines Zeitpunkts nach einer R-Welle, zu dem eine T-Welle zu erwarten wäre, und Setzen des sicheren Intervalls derart, dass es nach Ende der R-Welle startet und vor Beginn der T-Welle endet.

4. Ablationssystem nach Anspruch 1, wobei:

die Auslöseeinrichtung eine Detektionseinrichtung aufweist, die konfiguriert ist, einen Kardiozyklus zu detektieren und ein oder mehrere Merkmale des Kardiozyklus, darunter zumindest die R-Welle, zu identifizieren; und

die Analyseeinrichtung aufweist: eine Bestimmungseinrichtung zum Bestimmen einer Wartezeit von Beginn der R-Welle an bis zu einem sicheren Startzeitpunkt in dem Kardiozyklus und Bestimmen einer Zeitdauer von dem sicheren Startzeitpunkt zu einem Endzeitpunkt, während derer das sichere Zeitintervall definiert ist.

5. Ablationssystem nach Anspruch 4, wobei die Detektionseinrichtung konfiguriert ist, die T-Welle als eines der Merkmale des Kardiozyklus zu identifizieren; und die Bestimmungseinrichtung konfiguriert ist, den Endzeitpunkt als einen Zeitpunkt vor der T-Welle zu identifizieren.

6. Ablationssystem nach Anspruch 1, wobei die Auslöseeinrichtung aufweist:

eine Detektionseinrichtung, die konfiguriert ist, ein Kardiosignal zu detektieren und ein Ereignis in dem Kardiosignal zu identifizieren; und

eine Bestätigungseinrichtung zum Bestätigen des identifizierten Ereignisses in dem Kardiozyklus;

wobei die Auslöseeinrichtung konfiguriert ist, Auslösesignale nur als Antwort auf identifizierte, bestätigte Ereignisse auszugeben.

7. Ablationssystem nach Anspruch 6, wobei die Detektionseinrichtung konfiguriert ist, R-Wellen zu identifizieren, und die Bestätigungseinrichtung konfiguriert ist, das identifizierte Ereignis mit einem R-Welle-Muster zu vergleichen, um zu bestätigen, dass das identifizierte Ereignis eine R-Welle ist.

8. Ablationssystem nach Anspruch 6, wobei die Detektionseinrichtung konfiguriert ist, R-Wellen zu identifizieren, und die Bestätigungseinrichtung konfiguriert ist, eine Anstiegsrate des identifizierten Ereignisses zu berechnen und mit einer Schwelle zu vergleichen, um zu bestätigen, dass das identifizierte Ereignis eine R-Welle ist.

9. Ablationssystem nach Anspruch 6, ferner aufweisend einen Sensor zum Detektieren von Schall und/oder Druck und/oder Bewegung, wobei:

die Detektionseinrichtung konfiguriert ist, R-Wellen zu identifizieren; und

die Bestätigungseinrichtung konfiguriert ist, eine Ausgabe des Sensors mit dem identifizierten Ereignis zu korrelieren, um zu bestätigen, dass das identifizierte Ereignis eine R-Welle ist.

10. Ablationssystem nach Anspruch 6, wobei die Detektionseinrichtung konfiguriert ist, R-Wellen zu identifizieren, und die Bestätigungseinrichtung konfiguriert ist, eine Breite des identifizierten Ereignisses zu berechnen und die Breite mit einer Schwelle oder einem gespeicherten Wert zu vergleichen, um zu bestätigen, dass das identifizierte Ereignis eine R-Welle ist.

11. Ablationssystem nach Anspruch 6, wobei die Detektionseinrichtung konfiguriert ist, R-Wellen zu identifizieren, und die Bestätigungseinrichtung konfiguriert ist, eine Amplitude des identifizierten Ereignisses mit einer gespeicherten Amplitude einer vorherigen R-Welle zu vergleichen, um zu bestätigen, dass das identifizierte Ereignis eine R-Welle ist.

12. Ablationssystem nach Anspruch 6, wobei die Bestätigungseinrichtung konfiguriert ist, einen oder mehrere mit dem identifizierten Ereignis assoziierte Extrempunkte oder Wendepunkte in dem Kardiosignal zu identifizieren und zu zählen und mit einer Schwelle zu vergleichen, und irgendein Ereignis, das Extrempunkte oder Wendepunkte höher als die Schwelle hat, als Rauschen zu verwerfen.

13. Ablationssystem nach einem der vorstehenden Ansprüche, wobei die Auslöseeinrichtung konfiguriert ist, über eine Serie von Kardiozyklen hinweg zu arbeiten durch:

Auslösen von Ablation für einen oder mehrere erste Kardiozyklen unter Verwendung von ersten, das sichere Intervall definierenden Parametern;
Nicht-Auslösen von Ablation für einen oder mehrere zweite Kardiozyklen, die nach dem einen oder den mehreren ersten Kardiozyklen auftreten, und Betreiben der Analyseeinrichtung, um den einen oder die mehreren zweiten Kardiozyklen zu analysieren und zweite, das sichere Intervall definierende Parameter zu berechnen;
Auslösen von Ablation für einen oder mehrere dritte Kardiozyklen im Anschluss an die zweiten Kardiozyklen unter Verwendung der zweiten, das sichere Intervall definierenden Parameter.

14. Ablationssystem nach einem der Ansprüche 1 bis 13, ferner aufweisend eine Sonde zum Abgeben der Ausgabepulse aus den Pulserzeugungseinrichtungen an einen Patienten.

**Revendications**

1. Système d'ablation (18), comprenant :

un moyen de génération d'impulsions (130, 140) pour générer des impulsions de sortie à des fins d'ablation en réponse à un ou plusieurs signaux de déclenchement ;
un moyen de déclenchement (160) ayant un moyen d'entrée (164) pour recevoir des informations de signal cardiaque en provenance d'un patient et émettre des signaux de déclenchement pour l'émission d'une thérapie d'ablation ; et
un contrôleur de système (100) couplé à au moins chacun du moyen de génération d'impulsions et du moyen de déclenchement, dans lequel le moyen de déclenchement comprend un moyen d'analyse pour analyser les informations de signal cardiaque reçues et identifier des périodes de temps sûres pour l'émission d'une thérapie d'ablation, et dans lequel le moyen de déclenchement est configuré pour émettre les signaux de déclenchement de sorte que le moyen de génération d'impulsions génère des impulsions de sortie à des fins d'ablation pendant de telles périodes de temps sûres, **caractérisé en ce que**
le moyen d'analyse est configuré pour déterminer la période sûre en calculant un rythme cardiaque pour le patient et en ajustant un ou plusieurs éléments parmi un point de début pour la période sûre ou une durée de la période sûre, sur la base du rythme cardiaque calculé.

2. Système d'ablation selon la revendication 1, dans lequel le moyen de déclenchement est configuré pour faire fonctionner le moyen d'analyse afin d'analyser la temporisation de la période sûre après l'administration d'au moins une thérapie d'ablation dans le cadre d'un régime thérapeutique.

3. Système d'ablation selon la revendication 1, dans lequel le moyen de déclenchement comprend un moyen de détection pour détecter des cycles cardiaques et identifier une ou plusieurs ondes parmi des ondes R et des ondes T, dans lequel le moyen d'analyse est configuré pour régler la période sûre en estimant un temps après une onde R auquel une onde T serait attendue, et en réglant la période sûre afin qu'elle commence après la fin de l'onde R et qu'elle se termine avant le début de l'onde T.

4. Système d'ablation selon la revendication 1, dans lequel :

le moyen de déclenchement comprend un moyen de détection configuré pour détecter un cycle cardiaque et identifier une ou plusieurs caractéristiques du cycle cardiaque, y compris au moins l'onde R ; et
le moyen d'analyse comprend un moyen de détermination pour déterminer un délai entre l'apparition de l'onde R et un instant de début sûr dans le cycle cardiaque, ainsi qu'une période entre l'instant de début sûr et un instant de fin au cours duquel la période de temps sûre est définie.

5. Système d'ablation selon la revendication 4, dans lequel le moyen de détection est configuré pour identifier l'onde T comme étant l'une des caractéristiques du cycle cardiaque, et le moyen de détermination est configuré pour identifier l'instant de fin comme un instant antérieur à l'onde T.

6. Système d'ablation selon la revendication 1, dans lequel le moyen de déclenchement comprend :

un moyen de détection configuré pour détecter un signal cardiaque et identifier un événement dans le cycle cardiaque ; et
un moyen de confirmation pour confirmer l'événement identifié dans le cycle cardiaque ;
dans lequel le moyen de déclenchement est configuré pour n'émettre des signaux de déclenchement qu'en réponse à des événements identifiés et confirmés.

7. Système d'ablation selon la revendication 6, dans lequel le moyen de détection est configuré pour identifier des ondes R, et le moyen de confirmation est configuré pour comparer l'événement identifié à un modèle d'onde R afin de confirmer que l'événement identifié est une onde R.

8. Système d'ablation selon la revendication 6, dans lequel le moyen de détection est configuré pour identifier des ondes R, et le moyen de confirmation est configuré pour calculer la vitesse de balayage de l'événement identifié et pour la comparer à un seuil afin de confirmer que l'événement identifié est une onde R.

9. Système d'ablation selon la revendication 6, comprenant en outre un capteur pour détecter un ou plusieurs éléments parmi un son, une pression ou un mouvement, dans lequel :

le moyen de détection est configuré pour identifier des ondes R ; et
le moyen de confirmation est configuré pour corréler une sortie du capteur à l'événement identifié afin de confirmer que l'événement identifié est une onde R.

10. Système d'ablation selon la revendication 6, dans lequel le moyen de détection est configuré pour identifier des ondes R, et le moyen de confirmation est configuré pour calculer une largeur de l'événement identifié et pour comparer la largeur à un seuil ou à une valeur stockée afin de confirmer que l'événement identifié est une onde R.

11. Système d'ablation selon la revendication 6, dans lequel le moyen de détection est configuré pour identifier des ondes R, et le moyen de confirmation est configuré pour comparer une amplitude de l'événement identifié à une amplitude stockée d'une onde R précédente afin de confirmer que l'événement identifié est une onde R.

12. Système d'ablation selon la revendication 6, dans lequel le moyen de confirmation est configuré pour identifier et compter un ou plusieurs points parmi des points de basculement et des points d'inflexion dans le signal cardiaque associé à l'événement identifié, et pour les comparer à un seuil, et pour rejeter comme étant du bruit tout événement identifié ayant des points de basculement et des points d'inflexion supérieurs au seuil.

13. Système d'ablation selon l'une quelconque des revendications précédentes, dans lequel le moyen de déclenchement est configuré pour fonctionner sur une série de cycles cardiaques en :

déclenchant l'ablation pour un ou plusieurs premiers cycles cardiaques en utilisant des premiers paramètres définissant la période sûre ;
ne déclenchant pas l'ablation pour un ou plusieurs deuxièmes cycles cardiaques survenant après le ou les premiers cycles cardiaques, et en faisant fonctionner le moyen d'analyse pour analyser le ou les deuxièmes cycles cardiaques et calculer des seconds paramètres définissant la période sûre ;
déclenchant l'ablation pour un ou plusieurs troisièmes cycles cardiaques suivant les deuxièmes cycles cardiaques en utilisant les deuxièmes paramètres définissant la période sûre.

14. Système d'ablation selon l'une quelconque des revendications 1 à 13, comprenant en outre une sonde pour délivrer les impulsions de sortie, à partir du moyen de génération d'impulsions, à un patient.

FIG. 1

FIG. 2

FIG. 3

EP 4 138 695 B1

```
                                        ┌──────────────────┐╮300
                                        │  Start Regimen   │
                                        └──────────────────┘
                                                 ╷
                                                 ▼
                                        ┌──────────────────┐╮302
                                        │ Calculate Beat Rate │◄─┐
                                        └──────────────────┘  │
                                                 ╷            │
          ╮310                                   ▼            │
 ┌──────────────────┐              ┌──────────────────┐╮304   │
 │                  │              │ Set Delay Interval │      │
 │ ┌──────────────┐ │╮312          └──────────────────┘      │
 │ │ Beat Detect  │ │                       ╷                 │
 │ └──────────────┘ │                       ▼                 │
 │        ╷         │              ┌──────────────────┐╮306   │
 │        ▼         │              │  Deliver Therapy  │       │
 │ ┌──────────────┐ │╮314          └──────────────────┘      │
 │ │     Wait     │◄┼──────────             ╷                 │
 │ └──────────────┘ │                       ▼                 │
 │        ╷         │              ┌──────────────────┐╮320   │
 │        ▼         │              │ Regimen Complete? │──────┘
 │ ┌──────────────┐ │╮316          └──────────────────┘
 │ │    Issue     │ │                       ╷
 │ └──────────────┘ │                       ▼
 └──────────────────┘              ┌──────────────────┐╮322
                                   │       End        │
                                   └──────────────────┘
```

FIG. 4

EP 4 138 695 B1

```
356 ── Adjust Burst                    Start Regimen ──350

                                             │
                                             ▼
                                  Analyze Cardiac Beat ──352

                      360                    │
                       │                     ▼
              ┌───────────────────┐   Set Safe Window ──354
  362 ── │    Beat Detect    │
              │        │          │          │
              │        ▼          │          ▼
  364 ── │      Wait       │ ◄── Deliver Therapy ──358
              │        │          │
              │        ▼          │          │
  366 ── │     Issue      │          ▼
              └───────────────────┘   Regimen Complete? ──370

                                             │
                                             ▼
                                           End ──372
```

FIG. 5

FIG. 6

FIG. 7

FIG. 8

FIG. 9

FIG. 10

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10130819 B
- WO 2018005511 A1
- US 5855576 A
- US 6638277 B
- US 20190223943 A
- US 20200289185 A
- US 8926606 B
- US 8565878 B
- US 20090259271
- US 20100004713 A
- US 20110098585 A
- US 7248921 B